# EUROPEAN PATENT APPLICATION

(11) **EP 4 691 396 A1**
(43) Date of publication of application: **11.02.2026**
(21) Application number: 25194984.8
(22) Date of filing: 08.08.2025
(51) Int. Cl.: A61B 17/80

(54) **TIBIAL FIXATION PLATE**

(30) Priority: 08.08.2024 US 202418798236
(71) Applicant: Invictos LLC, Sturbridge, MA 01566 (US)
(72) Inventor: WOTTON, Harold, Sturbridge, 01566 (US)
(74) Representative: Dehns

(57) **Abstract**

A bone fixation plate (20) is provided that includes a body that extends between a proximal end (22) and a distal end (24). The body includes a top surface (30) and a bottom surface disposed opposite the top surface. The body includes a slotted aperture (160) extending between the top surface (30) and the bottom surface thereby forming a fastener passage therethrough. The slotted tail aperture (160) includes a length extending along a lengthwise axis and a width extending along a widthwise axis. The lengthwise axis extends between a first lengthwise end of the slotted aperture and a second lengthwise end of the slotted aperture (160). The second lengthwise end is opposite the first lengthwise end. The slotted aperture (160) includes a threaded first portion (160A) disposed at the first lengthwise end and a thread-free second portion (160B) disposed at the second lengthwise end.

## Description

### BACKGROUND OF THE INVENTION

### 1. Technical Field

The present invention relates generally to a bone fixation plate and, more particularly to a bone fixation plate configured to fix a tibia in animals, including canines.

### 2. Background Information

Bone fixation plates may be used in tibial osteotomy and other procedures to secure two bone segments together. In a tibial plateau leveling osteotomy (commonly referred to as "TPLO") procedure, for example, a curvilinear cut is made in the canine proximal tibia to separate the metaphysis from the proximal tibia. Next, the metaphysis is rotated to level the tibial plateau. Finally, the metaphysis is fixed to the proximal tibia by a fixation plate.

It is commonly desired by those performing a tibial osteotomy to make the curvilinear cut as proximally high and close to the joint between the tibia and the metaphysis as possible. However, conventional bone fixation plates do not allow such proximally high curvilinear cuts to occur. These proximally high curvilinear cuts are discouraged at least partially due to the large size of a head portion of a conventional bone fixation plate. Further, the large size of head portions and hole locations restricts the ability to manipulate the plate on the bone.

In addition, the geometry of conventional bone fixation plates is straight from a head of the plate to a tail of the plate. This restricts the purchase of the caudal aspect of the proximal tibia after rotation and the maximization of central coverage of the mid shaft tibia. Moreover, conventional bone fixation plates do not allow a place for implementation of a skin retractor. As a result, the incisions required when utilizing a conventional bone fixation plate are long.

Finally, conventional bone fixation plates have screw holes that are limited or restrictive as to how they allow screws inserted therein to be oriented. In other words, the conventional bone fixation plates do not allow for polyaxial placement of the screws.

The present invention provides an improved bone fixation plate allowing for a high proximal cut in the tibia while promoting caudal purchase of the plate on the proximal tibia and enabling a place for a skin retractor to engage the plate.

### SUMMARY OF THE INVENTION

According to an aspect of the present invention, a bone fixation plate is provided that includes a body that extends between a proximal end and a distal end. The body includes a top surface and a bottom surface disposed opposite the top surface. The body includes a slotted aperture extending between the top surface and the bottom surface thereby forming a fastener passage therethrough. The slotted tail aperture includes a length extending along a lengthwise axis and a width extending along a widthwise axis. The lengthwise axis extends between a first lengthwise end of the slotted aperture and a second lengthwise end of the slotted aperture. The second lengthwise end is opposite the first lengthwise end. The slotted aperture includes a threaded first portion disposed at the first lengthwise end and a thread-free second portion disposed at the second lengthwise end.

In any of the aspects or embodiments described above and herein, the length of the slotted aperture may be greater than the width of the slotted aperture.

In any of the aspects or embodiments described above and herein, the slotted aperture may have an open configuration between the first lengthwise end and the second lengthwise end.

In any of the aspects or embodiments described above and herein, the threaded first portion may have a central axis and may have a conical configuration.

In any of the aspects or embodiments described above and herein, the threaded first portion may have a central axis and a polyaxial configuration that includes a plurality of threaded sections with a relief pocket disposed between adjacent threaded sections.

In any of the aspects or embodiments described above and herein, the threaded sections may have a conical configuration.

In any of the aspects or embodiments described above and herein, the threaded first portion (TFP) may have a TFP central axis and the polyaxial configuration may be configured to permit a threaded fastener having a fastener central axis to be disposed in the threaded first portion with the fastener central axis parallel to the TFP central axis with threaded engagement between the threaded fastener and at least one of the plurality of threaded sections.

In any of the aspects or embodiments described above and herein, the threaded first portion (TFP) may have a TFP central axis and the polyaxial configuration may be configured to permit a threaded fastener having a fastener central axis to be disposed in the threaded first portion with the fastener central axis at a skewed angle with threaded engagement between the threaded fastener and at least one of the plurality of threaded sections.

In any of the aspects or embodiments described above and herein, the skewed angle may be up to and including fifteen degrees.

In any of the aspects or embodiments described above and herein, the body may include a head section, a tail section, and a curved section. The head section may optionally include a head section centerline that optionally bisects the head section into two substantially equal parts. The head section may be disposed at the proximal end, the tail section may be disposed at the distal end, and the curved section may be disposed between and contiguous with the tail section and the head section. The head section may optionally be oriented toward a caudal side of the body and the head section centerline may optionally be disposed at a head section angle, and the head section angle may be greater than zero.

In any of the aspects or embodiments described above and herein, the tail section may have a tail section centerline that extends along an arcuate path.

In any of the aspects or embodiments described above and herein: the body includes a head section disposed at the proximal end, a tail section disposed at the distal end, and a curved section disposed between and contiguous with the tail section and the head section; wherein the head section has a head section centerline that bisects the head section into two substantially equal parts; and wherein the head section centerline is substantially parallel to the lengthwise axis of the slotted aperture.

In any of the aspects or embodiments described above and herein, within the tail section, the body may include a caudal side surface extending between the top surface and the bottom surface, and a cranial side surface extending between the top surface and the bottom surface. The caudal side surface may be disposed on an inside of the arcuate path, and the cranial side surface may be disposed on an outside of the arcuate path.

In any of the aspects or embodiments described above and herein, the tail section may include a first tail aperture and a second tail aperture, each extending between the top surface and the bottom surface. The first tail aperture and the second tail aperture may each have an aperture central axis and a polyaxial configuration that includes a plurality of threaded sections with a relief pocket disposed between adjacent threaded sections. The first tail aperture may be disposed proximate the distal end of the body, and the second tail aperture may be disposed between the first tail aperture and the slotted aperture.

In any of the aspects or embodiments described above and herein, the polyaxial configuration of the first tail aperture and the second tail aperture may be configured to permit a respective threaded fastener having a fastener central axis to be disposed in the respective first tail aperture or second tail aperture at a skewed angle with threaded engagement between the threaded fastener and at least one of the plurality of threaded sections. The skewed angle may be up to and including fifteen degrees.

In any of the aspects or embodiments described above and herein, the curved section may curve caudally away from the tail section to project the head section caudally from the tail section.

In any of the aspects or embodiments described above and herein, the head section may include a head aperture having a head aperture central axis. The head aperture may have a polyaxial configuration that includes a plurality of threaded sections with a relief pocket disposed between adjacent threaded sections. The polyaxial configuration may permit a threaded fastener having a fastener central axis to be disposed in the head aperture at an angle such that the fastener central axis is skewed relative to the head aperture central axis, with threaded engagement between the threaded fastener and at least one of the plurality of threaded sections. The skewed angle may be up to and including fifteen degrees.

According to an aspect of the present invention, a bone fixation plate is provided that includes a head section and a tail section. The head section is disposed at a proximal end of the fixation plate. The head section has a first top surface, a first bottom surface opposite the first top surface, and at least one head aperture extending between the first top surface and the first bottom surface. The head section optionally has a head section centerline that bisects the head section. The tail section is disposed at a distal end of the fixation plate. The tail section includes a second top surface, a second bottom surface, at least one tail aperture, a slotted aperture, and a curved section. The second bottom surface is opposite the second top surface. The at least one tail aperture extends between the second top surface and the second bottom surface. The slotted aperture extends between the second top surface and the second bottom surface thereby forming a fastener passage through the head section. The slotted aperture includes a length extending along a lengthwise axis and a width extending along a widthwise axis. The lengthwise axis extends between a first lengthwise end of the slotted aperture and a second lengthwise end of the slotted aperture. The second lengthwise end is opposite the first lengthwise end. The slotted aperture includes a threaded first portion disposed at the first lengthwise end and a thread-free second portion disposed at the second lengthwise end. The curved section is disposed between and contiguous with the tail section and the head section. The head section is optionally oriented toward a caudal side of the fixation plate. The head section centerline is optionally disposed at a head section angle, and the head section angle is greater than zero.

The foregoing features and elements may be combined in various combinations without exclusivity, unless expressly indicated otherwise. These features and elements as well as the operation thereof will become more apparent in light of the following description and the accompanying drawings. It should be understood, however, the following description and drawings are intended to be exemplary in nature and non-limiting.

### BRIEF DESCRIPTION OF THE DRAWINGS

FIG. 1 is a perspective top view of a present disclosure fixation plate embodiment.
FIG. 2 is a planar top view of a present disclosure fixation plate embodiment.
FIG. 3 is the planar top view of a present disclosure fixation plate embodiment shown in FIG. 2, now including sectional cut lines.
FIG. 3A is a sectional view of the fixation plate shown in FIG. 3, along cut line A-A.
FIG. 3B is a sectional view of the fixation plate shown in FIG. 3, along cut line B-B.
FIG. 3C is a sectional view of the fixation plate shown in FIG. 3, along cut line C-C.
FIG. 4 is an enlarged partial view of the fixation plate shown in FIG. 3, including the slotted tail aperture.
FIG. 5 is a perspective bottom view of a present disclosure fixation plate embodiment.
FIG. 6 is a planar bottom view of a present disclosure fixation plate embodiment.
FIG. 7 is a planar side view of a present disclosure fixation plate embodiment.
FIG. 8 is a planar proximal end view of a present disclosure fixation plate embodiment.
FIG. 9 is the planar top view of a present disclosure fixation plate embodiment.
FIG. 10 is a partial sectional view along the sectional line 10-10 shown in FIG. 9.
FIG. 11 is an enlarged partial view of the fixation plate shown in FIG. 9, including the slotted tail aperture.
FIG. 12 is the planar top view of a present disclosure fracture plate embodiment.
FIG. 13 is the planar side view of the fracture plate embodiment shown in FIG. 12.

### DETAILED DESCRIPTION OF THE INVENTION

It is initially noted that, in the embodiments described herein, the standard anatomical terminology used corresponds to the standard anatomical position of a canine or an animal having hind limb anatomy corresponding with that of a canine. Application of the invention embodiments described herein is not restricted to a canine or an animal having hind limb anatomy corresponding with that of a canine. However, while it is contemplated that the invention could be applied to a human or an animal having lower leg anatomy corresponding with that of a human, the standard anatomical position of a human is different from the standard anatomical position of a canine. Thus, the standard anatomical terminology describing the application of the invention with respect to a canine or an animal having hind limb anatomy corresponding with that of a canine will not correspond to the standard anatomical terminology necessary to describe application of the invention with respect to a human or an animal having lower leg anatomy corresponding with that of a human.

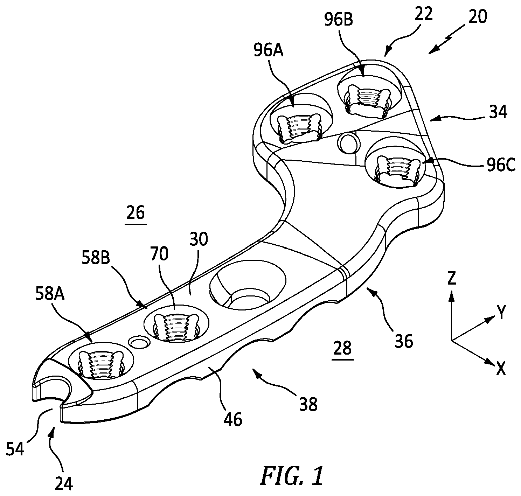

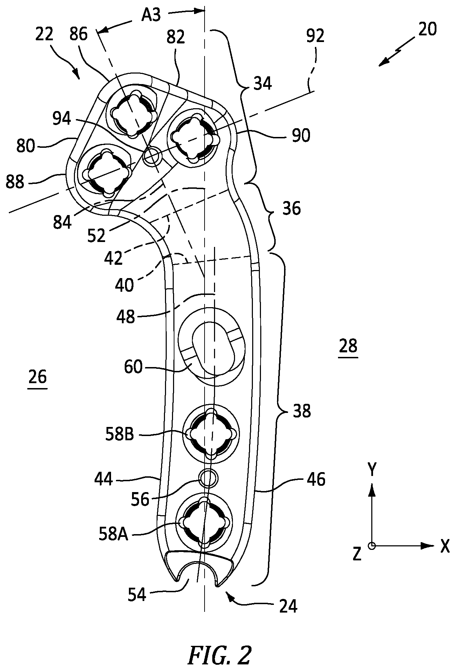

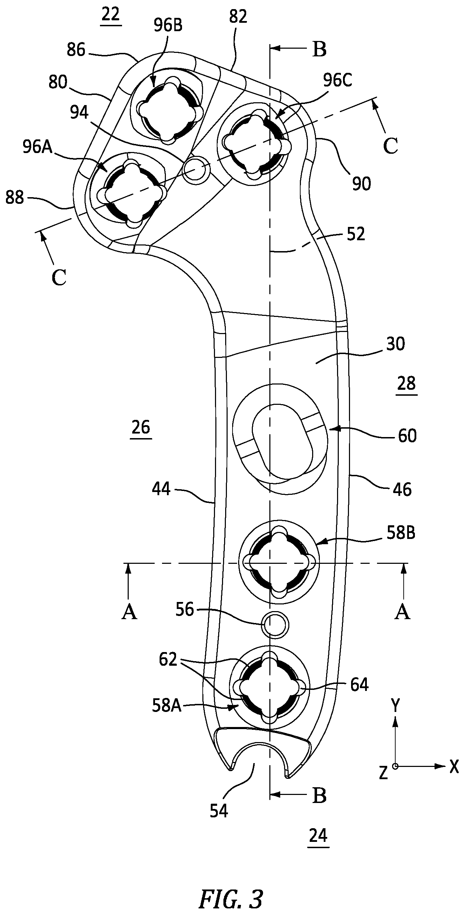

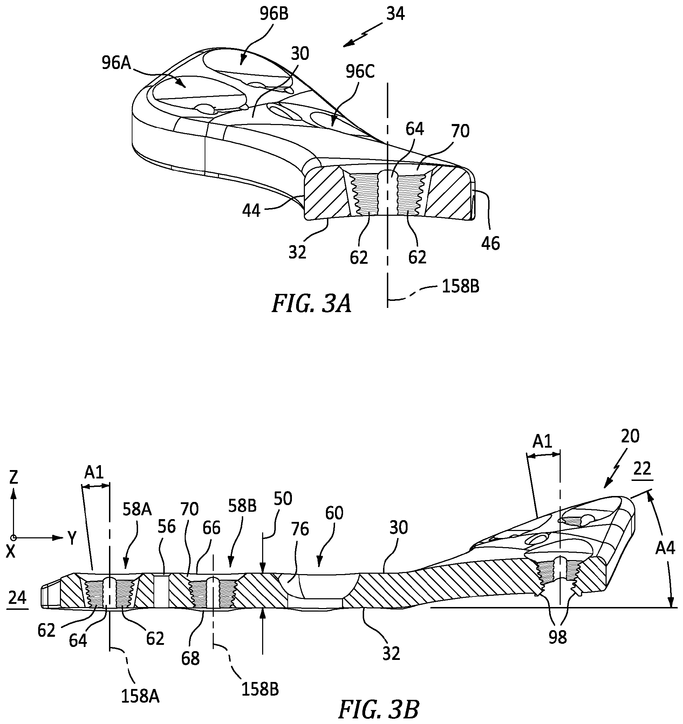

Referring to FIGS. 1-8, a tibial fixation plate 20 ("fixation plate 20") is diagrammatically shown that includes a proximal end 22, a distal end 24, a caudal side 26, a cranial side 28, a top surface 30, and a bottom surface 32 (e.g., see FIG. 3B). When in use, the bottom surface 32 generally faces the tibia. The top surface 30 is opposite the bottom surface 32. The fixation plate 20 may be described as having three distinct sections disposed longitudinally: a head section 34, a curved section 36, and a tail section 38. To facilitate the description herein, the fixation plate 20 may be described in terms of a body that includes the aforesaid three distinct sections (i.e., head section 34, curved section 36, tail section 38) disposed between the proximal end 22 and the distal end 24, and having the caudal side 26, the cranial side 28, the top surface 30, and the bottom surface 32. The head section 34 extends from the proximal end 22 of the fixation plate 20 toward the distal end 24 of the fixation plate 20. The tail section 38 extends from the distal end 24 of the fixation plate 20 toward the proximal end 22 of the fixation plate 20. The curved section 36 is disposed between and is contiguous with both the head section 34 and the tail section 38. A tail section - curved section boundary line 40 and a head section - curved section boundary line 42 are shown in FIG. 2 to illustrate the respective fixation plate 20 sections.

The tail section 38 of the fixation plate 20 is defined by the top surface 30, the bottom surface 32 (e.g., see FIG. 3B), a caudal edge surface 44, and a cranial edge surface 46. In a portion of the tail section 38, the caudal edge surface 44 and the cranial edge surface 46 may be disposed equidistant from one another. In some embodiments, the caudal edge surface 44 and the cranial edge surface 46 may converge toward one another at the distal end 24 of the fixation plate 20. In some embodiments, the tail section 38 may include a distal end surface that extends between the caudal edge surface 44 and the cranial edge surface 46 at the distal end 24 of the fixation plate 20. The fixation plate 20 embodiment shown in FIGS. 1-3 illustrates an example of a fixation plate 20 wherein the caudal edge surface 44 and the cranial edge surface 46 converge toward one another proximate the distal end 24. The present disclosure is not limited to this tail section 38 configuration.

Some of the FIGURES are shown with orthogonal axes X, Y, and Z to facilitate the description herein. The tail section 38 may be described as extending along a non-straight tail section centerline (TS centerline 48) that includes a lengthwise component (Y-axis direction) and a widthwise component (X-axis direction); e.g., the tail section may be described as itself being curved. The thickness 50 (see FIG. 3B) of the tail section 38 extends along the Z-axis (Z-axis perpendicular to the X-Y plane). The degree to which the tail section 38 may be non-straight (e.g., extending arcuately - curving - in the X-Y plane) may vary for particular embodiments, but is typically chosen to produce beneficial alignment between the tibia and the tail section 38. FIG. 2 illustrates a vertical line 52 (parallel to the Y-axis) that intersects with the center point of a first tail aperture 58A.

In the embodiment shown in FIGS. 1-3, for example, the tail section 38 extends along an arcuate path (i.e., TS centerline 48) that deviates from the vertical line 52 parallel to the Y-axis. Alternatively, the tail section 38 may extend along a non-straight path that includes compound curvature; e.g., a collection of arcuate subsections differing in curvature. As another example of a non-straight path, the tail section 38 may extend along a path that includes one or more arcuate subsections and one or more straight sections. These examples of a non-straight tail section path are provided to illustrate possible tail section centerline path configurations and are not intended to be limiting. The Y-axis component of the TS centerline 48 path is substantially greater than the X-axis component. The non-straight tail section 38 configuration of the tail section 38 is oriented such that caudal side 26 of the tail section 38 is the "inside" of the arcuate path and the cranial side 28 of the tail section 38 is the "outside" of the arcuate path. In the X-Y plane, the TS centerline 48 in the X-Y plane is equidistant from the caudal edge surface 44 and the cranial edge surface 46. The TS section centerline 48 may be configured as planar; e.g., the tail section 38 (and the TS centerline 48) extend in the X-Y plane without any curvature / deviation in the Z-axis direction (other than elements such as recessed channels in the tail section 38 as described herein.

FIGS. 1-3C illustrate an embodiment of the present disclosure fixation plate 20 that includes an open notch 54 disposed into the tail section 38 at the distal end 24. The open notch 54 may be configured to facilitate engagement of the fixation plate 20 with a surgical tool such as a Hohmann tissue retractor, or other surgical tool. The present disclosure does not require an open notch 54 disposed in the distal end 24 of the fixation plate 20.

The tail section 38 of the fixation plate 20 includes a tail positioning aperture 56, a first tail aperture 58A, a second tail aperture 58B, and a slotted tail aperture 60. The first tail aperture 58A is disposed adjacent the distal end 24 of the fixation plate 20 and the slotted tail aperture 60 is disposed adjacent the curved section 36. The second tail aperture 58B is disposed between the first tail aperture 58 and the slotted tail aperture 60. The first and second tail apertures 58A, 58B and the slotted tail aperture 60 may be centered on the TS centerline 48. The embodiment shown in FIGS. 1-3 shows the tail positioning aperture 56 disposed between the first and second tail apertures 58A, 58B. The present disclosure is not limited to locating the tail positioning aperture 56 between the first and second tail apertures 58A, 58B. The tail positioning aperture 56 is configured to receive a fixation member (not shown; e.g., a pin, screw, or other mechanical structure) for temporary positioning of the fixation plate 20 on the subject's tibia, and/or to facilitate a user visualizing a desired trajectory for fasteners to be inserted through the head apertures (described below). The tail apertures 58A, 58B enable the tail section 38 to be fixed to a bone segment; e.g., in a tibial plateau leveling osteotomy or "TPLO" procedure, the tail section 38 of the fixation plate 20 is attached to the distal / diaphysis segment of the tibia.

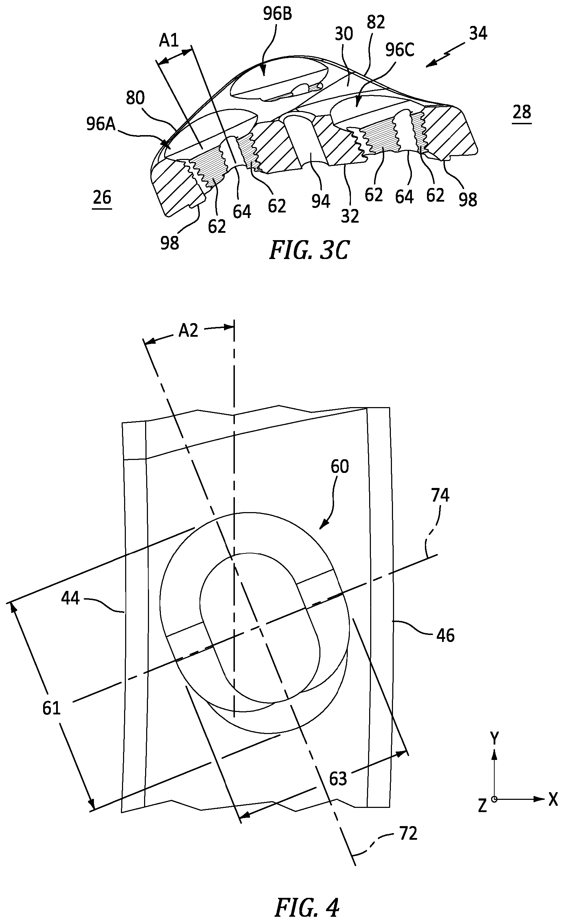

The first and second tail apertures 58A, 58B (and head apertures 96A-C as detailed below and shown in FIG. 3) each have a polyaxial configuration having a central axis 158A, 158B (e.g., see FIG. 3B) that extends through the thickness 50 of the tail section 38 (e.g., along the Z-axis). The polyaxial aperture configuration is understood to significantly enhance the clinician's ability to place a fastener into the bone in a desirable orientation. More specifically, the polyaxial configuration permits a fastener (e.g., a threaded screw) engaged within the aperture to be aligned with the central axis 158A, 158B of the aperture 58A, 58B (i.e., the central axis of the fastener is coincident with the central axis of the tail aperture) or disposed at an angle (e.g., angle A1 as shown in FIG. 3B) skewed from the central axis 158A 158B of the tail aperture 58A, 58B. In some embodiments, a polyaxial configuration as used in the present disclosure may permit a fastener to be engaged with a tail aperture 58A, 58B such that the central axis of the fastener is skewed at an angle (A1) of up to about fifteen degrees (15°) from the central axis 158A, 158B of the aperture 58A, 58B. Hence, the polyaxial configuration permits the user to orient a fastener in range of orientations (at any aperture circumferential position) and to select a preferred orientation for the application at hand.

Each threaded aperture with a polyaxial configuration may include a plurality of threaded sections 62 with adjacent threaded sections 62 are separated from one another by a relief pocket 64. In the fixation plate 20 example shown in the FIGURES, the first and second tail apertures 58A, 58B are shown having a polyaxial configuration with four (4) threaded sections 62 and four (4) relief pockets 64 (e.g., see FIG. 3). Each relief pocket 64 is disposed opposite (i.e., diagonally across through the center of the aperture) another of the relief pockets 64; e.g., the relief pockets 64 are disposed at ninety degree (90°) intervals around the circumference of the aperture 58A, 58B. The present disclosure is not limited to polyaxial configurations having four (4) threaded sections 62 and four (4) relief pockets 64, and may alternatively include more or less threaded sections 62 and relief pockets 64.

Each threaded tail aperture 58A, 58B has a first end 66 proximate the top surface 30 of the tail section 38 and a second end 68 proximate the bottom surface 32 of the tail section 38 (e.g., see FIG. 3B). In the embodiments shown in FIGS. 1-3C, the threaded sections 62 collectively form a conical configuration such that the threaded aperture 58A, 58B has a diameter at the first end 66 (proximate the top surface 30) that is greater than the diameter at the second end 68 (proximate the bottom surface 32). The conical configuration of the threaded sections / threaded aperture is understood to facilitate purposeful misalignment and yet still provide thread engagement between the threaded sections 62 and the fastener when fastener / aperture misalignment is desired. The present disclosure does not require threaded apertures 58A, 58B having a collective conical configuration.

In some embodiments, the first and/or second tail aperture 58A, 58B may be configured to receive at least a portion of the head of a fastener. In the embodiments shown in FIGS. 1-3C, for example, the first and second tail apertures include a countersink portion 70 disposed at the first end 66. The present disclosure does not require the first and/or second tail aperture 58A, 58B to include a countersink portion 70, or if included the countersink portion 70 is not limited to any particular configuration.

The present disclosure fixation plate 20 is not limited to using any particular type of fastener with the first and second tail apertures 58A, 58B. A fastener that includes a self-tapping tip and a threaded shank (including threads below a head of the fastener) is an example of a fastener that may be used. Specific types of fasteners that may be used include cortical screws and locking screws. The portion of the fastener threaded shank disposed adjacent the fastener head may include a thread form configured for threaded engagement with the thread sections of the respective first or second tail aperture 58A, 58B; e.g., a mating screw thread that avoids cross-threading. The entirety of the fastener shank (e.g., other than the self-tapping tip) may have the same thread configuration, or different portions of the fastener shank may have different thread configurations. The threaded engagement between the fastener and the threaded aperture 58A, 58B and the threaded engagement of the fastener with the bone permits positive positioning between the fixation plate 20 and the bone.

A first embodiment of the slotted tail aperture 60 is shown in FIGS. 1-4. As will be detailed herein, the present disclosure is not limited to this slotted tail aperture embodiment. In the embodiment shown in FIGS. 1-4, the slotted tail aperture 60 is generally oblong having a length 61 greater than a width 63 and is configured to receive a fastener. The length 61 of the slotted tail aperture 60 extends along a lengthwise axis 72. The width 63 of the slotted tail aperture 60 extends along a widthwise axis 74 perpendicular to the lengthwise axis 72. The slotted tail aperture 60 extends through the thickness 50 (see FIG. 3B) of the fixation plate 20 to enable a fastener to pass through the slotted tail aperture 60 to engage with the bone segment. The slotted tail aperture 60 is angularly disposed to enable the application of compression to the center of the osteotomy so that all or substantially all of the cut line is compressed. For example, the lengthwise axis 72 is disposed at an angle (A2 - see FIG. 4) relative to the Y-axis that may be substantially equal to the angle (A3 - see FIG. 2) at which the head section axis is disposed relative to the Y-axis as will be described below (e.g., A2 ≈ A3). In other words, the lengthwise axis 72 of the slotted tail aperture 60 may be disposed generally parallel to the head section axis. The length 61 of the slotted tail aperture 60 permits the user to vary placement of a fastener; e.g., to permit the user to position a fastener in a desired location; e.g., oriented more or less toward the cranial side 28 (or conversely the caudal side 26; e.g., see FIG. 3) of the tail section 38 and more or less toward the proximal end 22 (or conversely the distal end 24; e.g., see FIG. 3) of the fixture plate 20 and therefore relative to the curvilinear cut line.

As indicated above, in a TPLO procedure, a curvilinear cut is made in the canine proximal tibia to separate the metaphysis from the proximal tibia, thereby producing an arcuately shaped interface there between. The skewed angle A2 of the slotted tail aperture 60 lengthwise axis 72 is such that the lengthwise position of the fastener within the slotted tail aperture 60 does not appreciably change the orientation of the fastener toward or away from a lateral end of the arcuately shaped interface. Rather, the lengthwise position of a fastener within the slotted tail aperture 60 predominantly changes the position of the fastener relative to the central portion of the arcuately shaped interface. As a result, a fastener disposed in the slotted tail aperture 60 is understood to produce compression more desirably on the central portion of the arcuately shaped interface rather than on lateral end portions of the interface.

Embodiments of the present disclosure may include a slotted tail aperture 60 that is configured to receive a fastener without threaded engagement; i.e., in these embodiments the slotted tail aperture 60 is unthreaded. The slotted tail aperture 60 may be configured to receive at least a portion of the head of a fastener. In the embodiment shown in FIGS. 1-3C, for example, the slotted tail aperture 60 includes a countersink portion 76 (see FIG. 3B) disposed at the top surface 30 end of the slotted tail aperture 60. More specifically, the slotted tail aperture 60 shown in FIGS. 1-4 includes a relatively deep depth wise (Z-axis) countersink 76 that permits a fastener disposed within the slotted tail aperture 60 to be skewed relative to the lengthwise axis 72 and/or the widthwise axis 74 of the slotted tail aperture 60 in a variety of different angular orientations. The slotted tail aperture 60, including the countersink portion 76, may be configured to permit a variety of different fastener types (e.g., having different head configurations) to be used, and/or more than one fastener disposed within the slotted tail aperture 60 at a time. The present disclosure does not, however, require the slotted tail aperture 60 to include a countersink portion 76.

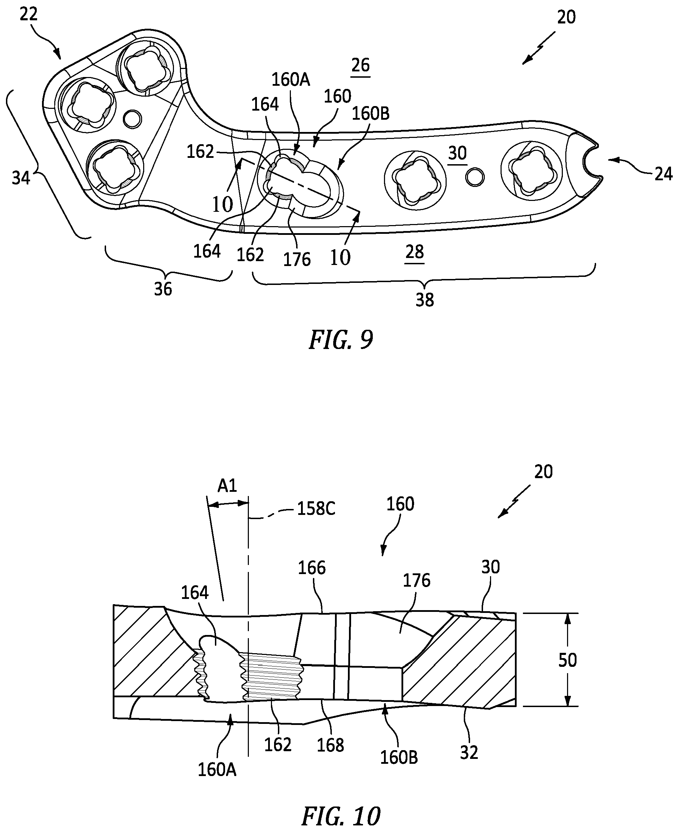

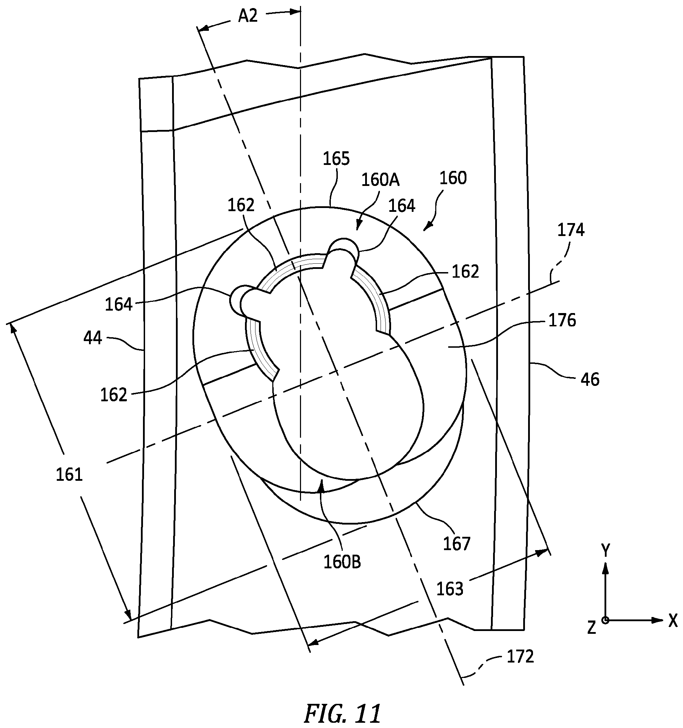

A second embodiment of the slotted tail aperture 160 is shown in FIGS. 9-11. This slotted tail aperture 160 embodiment may be configured similar to the slotted tail aperture 60 shown in FIGS. 1-4, with a generally oblong shape having a length 161 greater than a width 163 (e.g., see FIG. 11). The length 161 of the slotted tail aperture 160 extends along a lengthwise axis 172 between a first lengthwise end 165 and a second lengthwise end 167, opposite the first lengthwise end 165. The width 163 of the slotted tail aperture 160 extends along a widthwise axis 174 perpendicular to the lengthwise axis 172. The slotted tail aperture 160 extends through the thickness 50 (e.g., along the Z-axis - see FIG. 3B) of the fixation plate 20 to enable a fastener to pass through the slotted tail aperture 160 to engage with the bone segment. The slotted tail aperture 160 may be angularly disposed so that the lengthwise axis 172 is disposed at an angle (A2 - see FIG. 4) relative to the Y-axis that may be substantially equal to the angle (A3 - see FIG. 2) at which the head section axis 84 (e.g., see FIG. 2) is disposed relative to the Y-axis; e.g., A2 ≈ A3.

In this slotted tail aperture 160 embodiment, a first portion 160A of the slotted tail aperture 160 is threaded to enable engagement of the slotted tail aperture 160 with a threaded fastener and a second portion 160B of the slotted tail aperture 160 is thread-free to enable a fastener to be received within the second portion 160B of the slotted tail aperture 160. The first portion 160A of the slotted tail aperture 160 is disposed at one lengthwise end (e.g., the first lengthwise end 165) of the slotted tail aperture 160, and the second portion 160B of the slotted tail aperture 160 is disposed at the opposite lengthwise end (e.g., the second lengthwise end 167) of the slotted tail aperture 160. The first and second portions 160A, 160B of the slotted tail aperture 160 may intersect with one another to form an open configuration therebetween; e.g., the aperture 160 is continuous between the first and second lengthwise ends 165, 167. Depending on the configuration of the slotted tail aperture 160 (e.g., the length of the slotted tail aperture 160) and the position of the fastener within the slotted tail aperture 160, the open configuration between the first and second portions 160A, 160B may allow a portion of fastener engaged with first portion 160A to extend an amount into the second portion 160B, or vice versa.

In some embodiments, the threaded first portion 160A of the slotted tail aperture 160 may be configured with a polyaxial configuration like that described herein regarding first and second tail apertures 58A, 58B (e.g., see FIGS. 3 and 3B), having a central axis 158C that extends through the thickness 50 of the tail section 38 (e.g., along the Z-axis). In the polyaxial configuration, the threaded first portion 160A includes a plurality of threaded sections 162 with adjacent threaded sections 162 are separated from one another by a relief pocket 164. The second portion 160B of the slotted tail aperture 160 may be configured to provide a clearance fit with a fastener extending therethrough.

As indicated herein, the polyaxial configuration is understood to significantly enhance the clinician's ability to place a fastener into the bone in a desirable orientation; e.g., the rotational axis of a fastener engaged within the first threaded portion 160A having a polyaxial configuration may be aligned with, or skewed from, the central axis 158C of the threaded first portion 160A as described herein. In the slotted tail aperture 160 shown in FIGS. 9-11, the polyaxial configuration includes three (3) threaded sections 162 and two (2) relief pockets 164. The present disclosure is not limited to this polyaxial configuration; e.g., more or less threaded sections 62 and relief pockets 64 may be utilized.

The slotted tail aperture 160 has a first end 166 proximate the top surface 30 of the tail section 38 and a second end 168 proximate the bottom surface 32 of the tail section 38 (e.g., see FIG. 3B). In some embodiments, the threaded first portion 160A may have a conical configuration the same as or similar to that described herein with respect to threaded apertures 58A, 58B. The present disclosure does not require the threaded first portion 160A to have a conical configuration.

The slotted tail aperture 160 embodiment shown in FIGS 9-11 may be configured to receive at least a portion of the head of a fastener in the same manner or similar manner as described herein with respect to the slotted tail aperture 60 embodiment shown in FIGS. 1-3C; e.g., the slotted tail aperture 160 may include a countersink portion 176 disposed at the top surface 30 end of the slotted tail aperture 160. The slotted tail aperture 160 does not require a countersink portion 176, however.

The slotted tail aperture 160 embodiment shown in FIGS. 9-11 provides the clinician with the option of attaching the fixation plate 20 to the subject's bone using a fastener engaged with the threaded first section 160A of the slotted tail aperture 160, or engaged with the second section 160B of the slotted tail aperture 160 where the fastener forms a clearance fit with the slotted tail aperture 160. The anatomical configuration of tibias vary from subject to subject and that variance is relevant to the method of attachment of the fixation plate 20 to the subject's bone during a TPLO procedure. For example, in some instances the portion of the fixation plate 20 proximate the slotted tail aperture 160 may be in contact with the subject's bone and in other instances that portion of the fixation plate 20 may not be in contact with the subject's bone. The present disclosure slotted tail aperture 160 with a threaded first section 160A and a clearance fit second section 160B provides the clinician with attachment options that reflect the application at hand. For example, if the portion of the fixation plate 20 proximate the slotted tail aperture 160 is spaced apart from the subject's bone, a clinician may prefer to use a threaded screw engagement (via the threaded first section 160A) rather than a compression screw which may be subject to excessive stress in such an application. Conversely, if the portion of the fixation plate 20 proximate the slotted tail aperture 160 is in contact with the subject's bone, then a clinician may prefer to use a compression screw (via the threaded first section 160A) to achieve the benefits associated with compressing the TPLO curvilinear cut.

In some embodiments, the bottom surface 32 of the tail section 38 may be arcuately shaped. For example, in the embodiment shown in FIGS. 3A, 5, and 6, the bottom surface 32 of the tail section 38 is widthwise-curved; e.g., curved along the X-axis. In some embodiments, both the bottom and top surfaces 32, 30 of the tail section 38 may both be widthwise-curved. The arcuate shape of the bottom surface 32 may be chosen to complement a general tibial geometry.

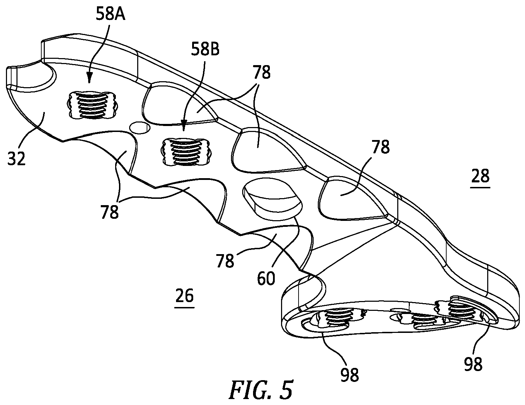

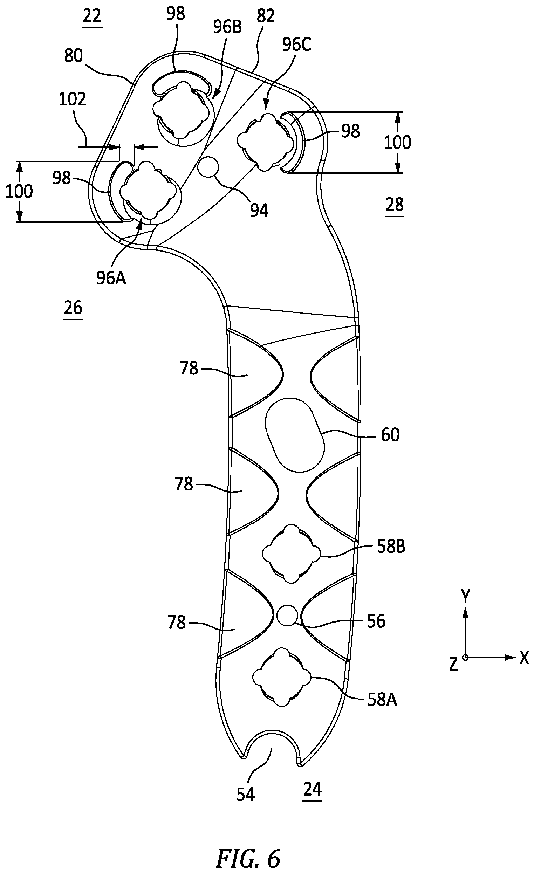

In some embodiments, the tail section 38 may include a plurality of recessed channels 78 intersecting the bottom surface 32 and the caudal edge surface 44, and a plurality of recessed channels 78 intersecting the bottom surface 32 and the cranial edge surface 46; e.g., see FIGS. 5 and 6. The recessed channels 78 decrease the surface area of the bottom surface 32 and thereby decrease the potential contact area between the bottom surface 32 of the fixation plate 20 and the tibia when the fixation plate 20 is fixed in place.

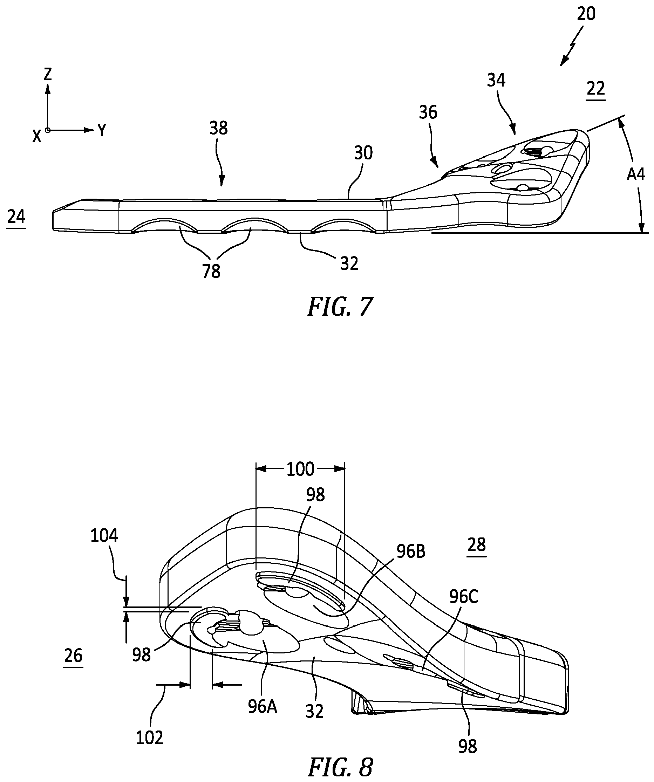

Referring to FIGS. 1-3, the head section 34 is disposed at the proximal end 22 of the fixation plate 20. The head section 34 is substantially diamond-shaped, defined at least in part by a first proximal edge surface 80 and a second proximal edge surface 82 that are oriented to intersect with one another. The head section 34 may be generally symmetric about a head section centerline 84 (HS centerline 84 - see FIG. 2). As will be described herein, embodiments of the head section 34 may assume a complex and/or an asymmetrical configuration. Hence, the statement that the head section 34 may be "generally symmetric" about the HS centerline 84 should not be interpreted as requiring the head section 34 to have perfectly symmetrical halves on opposite sides of the HS centerline 84. The head section 34 (and therefore the HS centerline 84) is disposed at an angle toward the caudal side 26 of the fixation plate 20. The HS centerline 84 bisects the head section 34 and is disposed at an angle A3 relative to the vertical line 52 (parallel to the Y-axis) shown intersecting the center point of the first tail aperture 58. The angle A3 at which the head section 34 is angularly disposed may be in the range of about fifteen degrees (15°) to about thirty-five degrees (35°). As can be seen in FIGS. 3B and 7, the head section 34 deviates from the X-Y plane in the Z-axis direction. FIG. 7 shows the head section 34 deviating by an angle A4. The angle A4 is shown in FIGS. 3B and 7 to illustrate the general angular deviation of the head section 34. The angle A4 at which the head section 34 is angularly disposed may be in the range of about twenty degrees (20°) to about forty-five degrees (45°). As will be described herein, the head section 34 has a complex curved shape and the degree of angular deviation (in the Z-axis direction) from the X-Y plane may vary across the head section 34; i.e., angle A4 may vary across the head section 34.

The head section 34 is anatomically contoured to reflect the contour of the tibia and to facilitate attachment of the fixation plate 20 to a portion of the tibia after rotation. The contouring includes the angular disposition of the head section 34 along the angles A3 and A4 and also may include a bottom surface 32 that is contoured to reflect the surface geometry of the tibia. The contouring of the head section 34 may be asymmetric. The head section 34 contour also allows the fixation plate 20 to be placed in multiple locations on the tibia to achieve the desired fixation after rotation. The shape of the head section 34 also allows for significant causal purchase in osteotomy. In preferred embodiments, the head section 34 is contoured to reflect an average contour of a tibia after a TPLO curvilinear cut is made and rotation occurs, based on a data base containing a clinically sufficient amount of empirical data.

In the embodiment shown in FIGS. 1-3, the first and second proximal edge surfaces 80, 82 are connected to one another by a connecting edge surface 86; e.g., see FIGS. 2 and 3. The HS centerline 84 bisecting the head section 34 may also bisect the connecting edge surface 86; e.g., see FIG. 2. The first proximal edge surface 80 transitions into the fixation plate curved section 36 via a first transition edge surface 88 and the second proximal edge surface 82 transitions into the fixation plate curved section 36 via a second transition edge surface 90. As illustrated in FIGS. 1-3, the connecting edge surface 86, the first transition edge surface 88, and the second transition edge surface 90 may be arcuately shaped. The width of the head section 34 may be described as extending along a line 92 perpendicular to the HS centerline 84. The width of the head section 34 may increase from the proximal end 22 of the fixation plate 20 (e.g., at the connecting edge surface 86) to a maximum (e.g., at about the first and third head apertures 96A, 96C) and then decrease to the curved section 36 of the fixation plate 20, thus producing the substantially diamond-shaped configuration. The head section 34 shown in FIGS. 1-3 is a non-limiting example of a head section 34 that may be used in the present disclosure fixation plate 20.

The head section 34 includes a head positioning aperture 94, a first head aperture 96A, a second head aperture 96B, and a third head aperture 96C. The head apertures 96A-C enable the head section 34 to be fixed to a tibial segment (e.g., the medial segment). The head positioning aperture 94 is configured to receive a fixation member (not shown; e.g., a pin, screw, or other mechanical structure) for temporary positioning of the fixation plate 20 on the subject's tibia, and/or to facilitate a user visualizing a desired trajectory for screws to be inserted through the head apertures. The first head aperture 96A is disposed on the caudal side 26 of the HS centerline 84 proximate a first lateral "point" of the diamond shape; e.g., see FIG. 2. The third head aperture 96C is disposed on the cranial side 28 of the HS centerline 84 proximate a second lateral "point" of the diamond shape, opposite the first lateral point. The head positioning aperture 94 may be disposed between the first and third head apertures 96A, 96C, generally aligned with the HS centerline 84. The present disclosure is not limited to locating the head positioning aperture 94 between the first and third head apertures 96A, 96C. The second head aperture 96B is disposed at the proximal end 22 of the fixation plate 20, generally aligned with the HS centerline 84, and centrally disposed relative to the first and third head apertures 96A, 96C albeit closer to the proximal end 22.

The first, second, and third head apertures 96A-C may have polyaxial configurations like those described above with respect to the first and second tail apertures 58A, 58B; e.g., a polyaxial configuration that permits a fastener engaged within the head aperture 96A-C to be aligned with the central axis of the head aperture 96A-C or disposed at an angle skewed from the central axis of the head aperture 96A-C. The polyaxial configuration includes a plurality of threaded sections 62 with adjacent threaded sections 62 that are separated from one another by a relief pocket 64 (e.g., as described herein with respect to first and second tail apertures 58A, 58B), and the threaded sections 62 may collectively form a conical configuration, etc. Each of the head apertures 96A-C may also be configured to receive at least a portion of the head of a fastener in the manner described above with respect to the tail apertures 58A, 58B.

Referring to FIGS. 3B, 3C, 5, 6, and 8, in some embodiments of the present disclosure one or more screw angle restriction projections 98 ("SAR projections 98") may be disposed proximate any or all of the first, second, and third head apertures, extending out from the bottom surface 32 of the head section 34. As described above, the polyaxial configuration of the head apertures 96A-C permits a fastener (e.g., a threaded screw) engaged within the head aperture 96A-C to be disposed at an angle A1 (e.g., see FIG. 3B) skewed from the central axis of the head aperture 96A-C, and the angle A1 may be up to about fifteen degrees (15°) from the central axis of the head aperture 96A-C. The SAR projections 98 are configured to limit the magnitude of the angle (A1) at which the fastener central axis may be skewed from the central axis of the head aperture 96A-C in predetermined regions of the respective head aperture 96A-C. In other words, each SAR projection 98 is disposed to limit the fastener skew angle in a predetermined region of the respective head aperture 96A-C. In those regions of a head aperture 96A-C void of a SAR projection 98, the fastener may be skewed at the otherwise possible skew angle A1. The SAR projections 98 function to prevent a fastener being inserted into a region of a tibia portion where fastener incursion may be problematic; e.g., in a direction toward the joint with the femur.

A SAR projection 98 includes a circumferentially extending length 100, a width 102, and a height 104; e.g., see FIGS. 6 and 8. The circumferential length 100 extends a predetermined portion of the outer diameter of the respective head aperture 96A-C. The circumferential length 100 may be chosen based on the region of the tibia that it is preferential to avoid skewed fastener placement completely, or a skewed fastener placement beyond a predetermined skew angle. For example, in the region of the head aperture where the SAR projection 98 is disposed, a SAR projection 98 may be configured to prevent any fastener skew; i.e., in the region of the head aperture 96A-C where the SAR projection 98 is disposed the fastener may only be oriented with its central axis coinciding with the central axis of the respective head aperture 96A-C. Alternatively, in the region of the head aperture 96A-C where the SAR projection 98 is disposed, a SAR projection 98 may be configured to permit limited fastener skew; i.e., in the region of the head aperture 96A-C where the SAR projection 98 is disposed the fastener may be permitted to be oriented with its central axis skewed a percentage of the maximum permitted fastener skew angle possible elsewhere around the head aperture 96A-C where the SAR projection 98 is not present (e.g., 50% of A1, or only about 7.5 degrees of skew if the maximum permitted skew angle elsewhere is 15 degrees, or the like).

In the example embodiment shown in FIGS. 3C, 5, 6, and 8, the SAR projection 98 associated with the first head aperture 96A is disposed on the caudal side 26 of the first head aperture 96A, the SAR projection 98 associated with the second head aperture 96B is disposed on the proximal end 22 side of the second head aperture 96B (see FIG. 6), and the SAR projection 98 associated with the third head aperture 96C is disposed on the cranial side 28 of the third head aperture 96C. In this example embodiment, each of these SAR projections 98 has a length 100 of about one third of the circumference of the respective head aperture circumference at the bottom surface 32. In this example embodiment, each of these SAR projections 98 has a height 104 sufficient to limit the fastener skew angle to that desired in that region; e.g., a range of A1 less than maximum, including A1 = 0.

The configuration of the SAR projections 98 shown in FIGS. 3C, 5, 6, and 8 (e.g., circumferentially extending length, width and height) are an example of a SAR projection 98 configuration and the present disclosure is not limited thereto. The positioning of the SAR projections 98 shown in FIGS. 3C, 5, 6, and 8 is an example of SAR projection 98 positioning, and the present disclosure is not limited thereto. The SAR projection 98 examples shown in FIGS. 3C, 5, 6, and 8 include a single SAR projection 98 per head aperture 96A-C. In alternative embodiments, the present disclosure may include more than one SAR projection 98 per head aperture 96A-C. The SAR projection 98 examples are described above as being associated with head aperture 96A-C. In some embodiments, the present disclosure may include one or more SAR projections 98 associated with tail apertures 58A, 58B.

As stated above, the curved section 36 of the fixation plate 20 is disposed between and is contiguous with both the head section 34 and the tail section 38. The tail section - curved section boundary line 40 and the head section - curved section boundary line 42 shown in FIG. 2 are included to illustrate the approximate boundaries between the respective fixation plate sections 34, 36, 38. The curved section 36 curves caudally away from tail section 38. The curvature of the curved section 36 in the X-Y plane is typically chosen to accommodate the desired orientation of the head section 34 described above (e.g., a HS centerline angle A3 - see FIG. 2). The curved section 36 may also deviate in the Z-axis direction from the X-Y plane of the tail section 38, here again to accommodate the desired orientation of the head section 34 described above (e.g., a HS centerline angle A4 - see FIG. 3B).

The fixation plate 20 may comprise a variety of different materials. For example, in some embodiments, a fixation plate 20 may comprise surgical implant grade 316L stainless steel, or titanium, or a polymeric material (e.g., a bioabsorbable polymer), or the like, or any combination thereof. In some embodiments, a fixation plate 20 may include one or more materials that support and stimulate bone growth, or pharmaceuticals or other healing compounds, or the like, and any combination thereof. In some embodiments, a fixation plate 20 may include a radiolucent material to enable only the bone growth to be seen when x-rayed, but not fixation plate 20 itself.

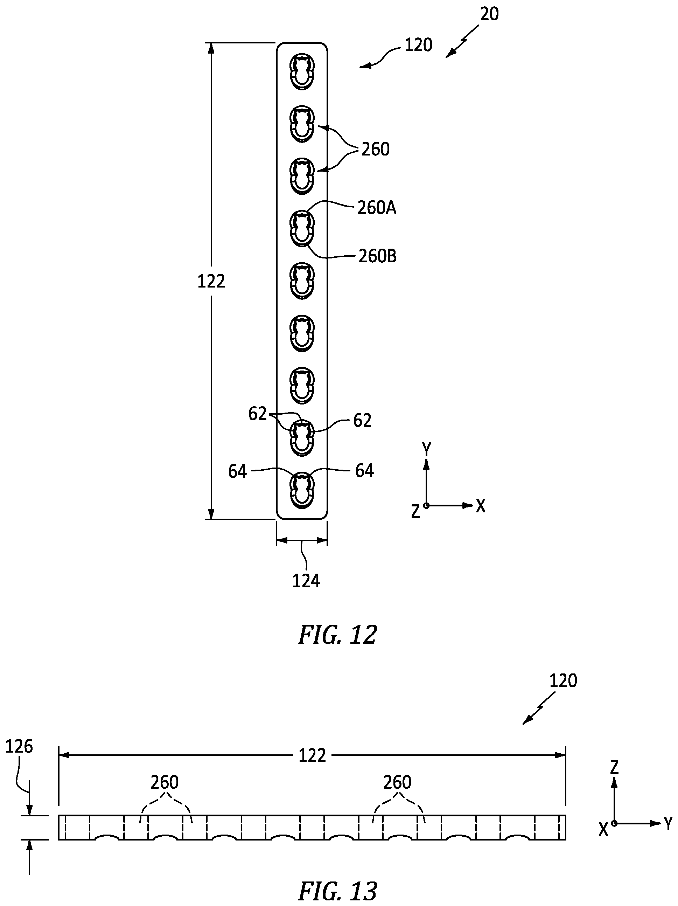

The present disclosure is not limited to using the polyaxial configuration described herein on fixation plates 20 utilized in a TPLO procedure. Referring to FIGS. 12 and 13, a fracture plate 120 (i.e., an alternative type of fixation plate 20) is diagrammatically illustrated that includes a length 122 (Y-axis), a width 124 (X-axis), and a thickness 126 (Z-axis), and a plurality of slotted apertures 260, each slotted aperture 260 having a first portion 260A with a polyaxial configuration and a second portion 260B the same or substantially the same as the slotted tail apertures 160 described herein with a threaded first portion 160A and as second portion 160B as described herein (e.g., see FIGS. 9-11). In this example, all of the apertures shown in the fracture plate 120 are shown as slotted apertures 260 having a polyaxial configuration but that is not required. As indicated herein, the rotational axis of a fastener engaged within a slotted aperture first portion 260A having a polyaxial configuration may be aligned with, or skewed from, the central axis of the fastener aperture. Each slotted aperture 260 with a polyaxial configuration may include a plurality of threaded sections 62 with adjacent threaded sections 62 are separated from one another by a relief pocket 64; e.g., a configuration like that shown in FIGS. 3A-C. The present disclosure is not limited to the fracture plate configuration of a fixation plate 20 shown in FIGS. 12 and 13, and the present disclosure slotted apertures 260 with a first portion 260A having a polyaxial configuration may be utilized on other types of fixation plates.

While the principles of the disclosure have been described above in connection with specific apparatuses and methods, it is to be clearly understood that this description is made only by way of example and not as limitation on the scope of the disclosure.

The singular forms "a," "an," and "the" refer to one or more than one, unless the context clearly dictates otherwise. For example, the term "comprising a specimen" includes single or plural specimens and is considered equivalent to the phrase "comprising at least one specimen." The term "or" refers to a single element of stated alternative elements or a combination of two or more elements unless the context clearly indicates otherwise. As used herein, "comprises" means "includes." Thus, "comprising A or B," means "including A or B, or A and B," without excluding additional elements. Further, the term "coupled" does not exclude the presence of intermediate elements between the coupled items. Also, any reference to attached, fixed, connected or the like may include permanent, removable, temporary, partial, full and/or any other possible attachment option.

It is noted that various connections are set forth between elements in the present description and drawings (the contents of which are included in this disclosure by way of reference). It is noted that these connections are general and, unless specified otherwise, may be direct or indirect and that this specification is not intended to be limiting in this respect. A coupling between two or more entities may refer to a direct connection or an indirect connection. An indirect connection may incorporate one or more intervening entities or a space/gap between the entities that are being coupled to one another.

Furthermore, no element, component, or method step in the present disclosure is intended to be dedicated to the public regardless of whether the element, component, or method step is explicitly recited in the claims.

While various inventive aspects, concepts and features of the disclosures may be described and illustrated herein as embodied in combination in the exemplary embodiments, these various aspects, concepts, and features may be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures--such as alternative materials, structures, configurations, methods, devices, and components, alternatives as to form, fit, and function, and so on--may be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein. For example, in the exemplary embodiments described above within the Detailed Description portion of the present specification, elements are described as individual units and shown as independent of one another to facilitate the description. In alternative embodiments, such elements may be configured as combined elements.

Additionally, even though some features, concepts, or aspects of the disclosures may be described herein as being a preferred arrangement or method, such description is not intended to suggest that such feature is required or necessary unless expressly so stated. Still further, exemplary or representative values and ranges may be included to assist in understanding the present application, however, such values and ranges are not to be construed in a limiting sense and are intended to be critical values or ranges only if so expressly stated.

Descriptions of exemplary methods or processes are not limited to inclusion of all steps as being required in all cases, nor is the order that the steps are presented to be construed as required or necessary unless expressly so stated. The words used in the claims have their full ordinary meanings and are not limited in any way by the description of the embodiments in the specification.

## Claims

1. A bone fixation plate, comprising:
a body extending between a proximal end and a distal end, the body including a top surface and a bottom surface disposed opposite the top surface;
wherein the body includes a slotted aperture extending between the top surface and the bottom surface thereby forming a fastener passage therethrough, wherein the slotted aperture includes a length extending along a lengthwise axis and a width extending along a widthwise axis, wherein the lengthwise axis extends between a first lengthwise end of the slotted aperture and a second lengthwise end of the slotted aperture, wherein the second lengthwise end is opposite the first lengthwise end;
wherein the slotted aperture includes a threaded first portion disposed at the first lengthwise end and a thread-free second portion disposed at the second lengthwise end.

2. The bone fixation plate of claim 1, wherein:
the slotted aperture has an open configuration between the first lengthwise end and the second lengthwise end; and/or
the length of the slotted aperture is greater than the width of the slotted aperture.

3. The bone fixation plate of claim 1 or 2, wherein the threaded first portion has a central axis and has a conical configuration.

4. The bone fixation plate of any preceding claim, wherein the threaded first portion has a or the central axis and a polyaxial configuration that includes a plurality of threaded sections with a relief pocket disposed between adjacent threaded sections,
optionally wherein the threaded sections have a conical configuration.

5. The bone fixation plate of claim 4, wherein the threaded first portion (TFP) has a TFP central axis and the polyaxial configuration is configured to permit a threaded fastener having a fastener central axis to be disposed in the threaded first portion with the fastener central axis parallel to the TFP central axis with threaded engagement between the threaded fastener and at least one of the plurality of threaded sections.

6. The bone fixation plate of claim 4 or 5, wherein the threaded first portion (TFP) has a or the TFP central axis and the polyaxial configuration is configured to permit a or the threaded fastener having a or the fastener central axis to be disposed in the threaded first portion with the fastener central axis at a skewed angle from the TFP central axis with threaded engagement between the threaded fastener and at least one of the plurality of threaded sections,
optionally wherein the skewed angle is up to and including fifteen degrees.

7. The bone fixation plate of any preceding claim, wherein:
the body includes a head section, a tail section, and a curved section; and
the head section is disposed at the proximal end, the tail section is disposed at the distal end, and the curved section is disposed between and contiguous with the tail section and the head section,
optionally wherein:
the head section includes a head section centerline;
the head section is oriented toward a caudal side of the body and the head section centerline is disposed at a head section angle, and the head section angle is greater than zero.

8. The bone fixation plate of any one of claims 1-6, wherein:
the body includes a head section disposed at the proximal end, a tail section disposed at the distal end, and a curved section disposed between and contiguous with the tail section and the head section;
wherein the head section has a head section centerline that bisects the head section into two substantially equal parts; and
wherein the head section centerline is substantially parallel to the lengthwise axis of the slotted aperture.

9. The bone fixation plate of claim 7 or 8, wherein the tail section has a tail section centerline that extends along an arcuate path.

10. The bone fixation plate of claim 9, wherein:
within the tail section, the body includes a caudal side surface extending between the top surface and the bottom surface, and a cranial side surface extending between the top surface and the bottom surface; and
the caudal side surface is disposed on an inside of the arcuate path, and the cranial side surface is disposed on an outside of the arcuate path.

11. The bone fixation plate of claim 9 or 10, wherein:
the tail section includes a first tail aperture and a second tail aperture, each extending between the top surface and the bottom surface;
the first tail aperture and the second tail aperture each have an aperture central axis and a polyaxial configuration that includes a plurality of threaded sections with a relief pocket disposed between adjacent threaded sections; and
the first tail aperture is disposed proximate the distal end of the body, and the second tail aperture is disposed between the first tail aperture and the slotted aperture.

12. The bone fixation plate of claim 11, wherein the polyaxial configuration of the first tail aperture and the second tail aperture is configured to permit a respective threaded fastener having a fastener central axis to be disposed in the respective first tail aperture or second tail aperture at a skewed angle from the aperture central axis with threaded engagement between the threaded fastener and at least one of the plurality of threaded sections, wherein the skewed angle is up to and including fifteen degrees.

13. The bone fixation plate of any of claims 7 to 12, wherein the curved section curves caudally away from the tail section to project the head section caudally from the tail section.

14. The bone fixation plate of any of claims 7 to 13, wherein the head section includes a head aperture having a head aperture central axis, the head aperture having a polyaxial configuration that includes a plurality of threaded sections with a relief pocket disposed between adjacent threaded sections, the polyaxial configuration permitting a threaded fastener having a fastener central axis to be disposed in the head aperture at an angle such that the fastener central axis is skewed relative to the head aperture central axis, with threaded engagement between the threaded fastener and at least one of the plurality of threaded sections, wherein the skewed angle is up to and including fifteen degrees.

15. A bone fixation plate, comprising:
a head section disposed at a proximal end of the fixation plate, the head section having a first top surface, a first bottom surface opposite the first top surface, and at least one head aperture extending between the first top surface and the first bottom surface;
a tail section disposed at a distal end of the fixation plate, the tail section having:
a second top surface;
a second bottom surface opposite the second top surface;
at least one tail aperture extending between the second top surface and the second bottom surface; and
a slotted aperture extending between the second top surface and the second bottom surface thereby forming a fastener passage through the tail section, wherein the slotted aperture includes a length extending along a lengthwise axis and a width extending along a widthwise axis, wherein the lengthwise axis extends between a first lengthwise end of the slotted aperture and a second lengthwise end of the slotted aperture, wherein the second lengthwise end is opposite the first lengthwise end, and wherein the slotted aperture includes a threaded first portion disposed at the first lengthwise end and a thread-free second portion disposed at the second lengthwise end; and
a curved section disposed between and contiguous with the tail section and the head section,
optionally wherein:
the head section has a head section centerline that bisects the head section; and
the head section is oriented toward a caudal side of the fixation plate and the head section centerline is disposed at a head section angle, and the head section angle is greater than zero.
